**Europäisches Patentamt**

**European Patent Office**

**Office européen des brevets**

(19)

(11) Veröffentlichungsnummer: **0 163 674**
**B1**

(12) # EUROPÄISCHE PATENTSCHRIFT

(45) Veröffentlichungstag der Patentschrift:
**23.08.89**

(51) Int. Cl.⁴: **A 61 B 3/02**, A 61 B 5/16

(21) Anmeldenummer: **84904107.4**

(22) Anmeldetag: **15.11.84**

(86) Internationale Anmeldenummer:
**PCT/DE 84/00246**

(87) Internationale Veröffentlichungsnummer:
**WO 85/02103 (23.05.85 Gazette 85/12)**

(54) **VORRICHTUNG ZUR GESICHTSFELDPRÜFUNG (III).**

(30) Priorität: **15.11.83 DE 3341395**

(43) Veröffentlichungstag der Anmeldung:
**11.12.85 Patentblatt 85/50**

(45) Bekanntmachung des Hinweises auf die Patenterteilung:
**23.08.89 Patentblatt 89/34**

(84) Benannte Vertragsstaaten:
**AT BE CH DE FR GB LI LU NL SE**

(56) Entgegenhaltungen:
**DE-C-3 135 383**
**DE-C-3 135 384**
**US-A-3 288 546**
**US-A-3 421 498**
**US-A-4 146 311**
**US-A-4 255 022**

**Proceedings of the Institution of Electrical Engineers, vol. 120, no. 11, November 1973, Stevange/Herts (GB). G.B.B. Chaplin et al.: "Automated system for testing visual fields", pages 1321-1327**

(73) Patentinhaber: **Optische Werke G. Rodenstock, Isartalstrasse 43, D-8000 München 5 (DE)**

(72) Erfinder: **WIECZOREK, Hannelore, Kandinsky- Str. 27, D-8000 München 71 (DE)**

(74) Vertreter: **Steinmann, Otto C., Kanzlei Münich, Steinmann, Schiller Willibaldstrasse 36, D-8000 München 21 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 163 674 B1

LIBER, STOCKHOLM 1989

**Beschreibung**

Die Erfindung bezieht sich auf eine Vorrichtung zur Gesichtsfeldprüfung gemäß dem Oberbegriff des Patentanspruchs 1.

Eine derartige Vorrichtung zur Gesichtsfeldprüfung ist beispielsweise aus der DE-A-3 135 384 (Rodenstock) bekannt. Auf den Inhalt dieser Druckschrift sowie auf den Inhalt der DE-A-3 135 383 (Rodenstock), in der eine Vorrichtung zur Gesichtsfeldprüfung abweichender Gattung beschrieben wird, wird im übrigen hinsichtlich der Erläuterung von in dieser Anmeldung verwendeten Begriffen sowie des allgemeinen Aufbaus und des weiteren Standes der Technik ausdrücklich Bezug genommen wird.

Bei den in diesen Druckschriften beschriebenen Vorrichtungen sollen die Intensität der aufleuchtenden Lichtpunkte in Abhängigkeit von der Defektquote bzw. die Intervallzeit in Abhängigkeit von der Reaktionszeit gesteuert werden.

Beispielsweise werden bei der in der DE-A-3 135 383 beschriebenen Vorrichtung die Intensität der dargebotenen Lichtpunkte entsprechend der Defektquote, d.h. der Lichtpunkte, die die zu untersuchende Person bei einer der Empfindlichkeit des Auges angepaßten Intensität nicht sieht, erhöht, und verschiedene Lichtpunkte mehrfach abgefragt in Abhängigkeit davon, ob sie bei erhöhter Intensität gesehen oder nicht gesehen werden. Dabei kann bei der in der DE-A-3 135 384 beschriebenen Vorrichtung die Intervallzeit - d.h. die Zeit zwischen dem Ende des Aufleuchtens des letzten Lichtpunktes (ein aufleuchtender Lichtpunkt wird im folgenden auch als Lichtreiz bezeichnet) und dem Beginn der nächsten Darbietung, die z. B. ein akustisches Aufmerksamkeitssignal für die zu untersuchende Person oder der Lichtreiz selbst sein kann - vergrößert oder verkleinert werden, in Abhängigkeit davon, ob die Reaktionszeit der zu untersuchenden Person zu- oder abnimmt. Hierbei wird unter Reaktionszeit die Zeit verstanden, die zwischen dem Aufleuchten eines Lichtpunktes und der Antwort "gesehen" verstreicht, die beispielsweise durch Drücken einer Taste in die Steuereinheit eingegeben wird.

Es hat sich nun herausgestellt, daß gerade bei schwierigen Patienten, wie Kindern, älteren Personen etc. die Abnahme des Konzentrationsvermögens während der Untersuchung einen großen Einfluß auf das Untersuchungsergebnis hat.

Weiterhin führt der Einsatz von ungeübtem Bedienungspersonal unter Umständen zu Schwierigkeiten, da das Bedienungspersonal Entscheidungen beispielsweise über die zu wählende "Untersuchungsstrategie" zu treffen hat, und durch falsche Entscheidungen die zu untersuchende Person bei der Untersuchung überfordert. Eine Überforderung hat aber zur Folge, daß bei der Gesichtsfeldprüfung nicht das wahre Sehvermögen ermittelt wird.

Der Erfindung liegt die Aufgabe zugrunde, eine Vorrichtung zur Gesichtsfeldprüfung gemäß dem Oberbegriff des Patentanspruchs 1 derart weiterzubilden, daß Verfälschungen des Untersuchungsergebnisses durch die Abnahme des Konzentrationsvermögens vermieden werden.

Diese Aufgabe wird erfindungsgemäß durch die im kennzeichnenden Teil des Patentanspruchs 1 angegebenen Merkmale gelöst. Erfindungsgemäß hat es sich herausgestellt, daß die Veränderung der Reaktionszeit ein gutes Maß für das Konzentrationsvermögen ist. Deshalb steuert die Steuereinheit die Darbietung der Lichtreize, d.h. das Aufleuchten der verschiedenen über das Gesichtsfeld verteilten Lichtpunkte in Abhängigkeit von der Reaktionszeit der zu untersuchenden Person.

Insbesondere übernimmt die Steuereinheit die während einer Untersuchung zu treffenden Entscheidungen; beispielsweise entscheidet die Steuereinheit, welche Gesichtsfeldbereiche untersucht werden sollen, ob die Defekte im Gesichtsfeld durch Variation der Intensität der dargebotenen Lichtpunkte ausgelotet werden sollen, oder ob der Patient noch in der Lage ist der Darbietung von Lichtpunkten zu folgen.

Das Überschreiten eines bestimmten Grenzwertes der Reaktionszeit wird erfindungsgemäß von der Steuereinheit als Kriterium für das Auftreten von Ermüdungserscheinungen herangezogen. Die Steuereinheit führt daraufhin die Untersuchung des Areals noch fort, das im Zeitpunkt des Überschreitens der Reaktionszeit untersucht worden ist, und beendet anschließend den Untersuchungsvorgang.

Der Grenzwert für die Reaktionszeit kann dabei fallbezogen festgelegt werden, z. B. kann er bei Kindern kleiner gewählt werden als bei alten Personen. Besonders vorteilhaft ist es jedoch, den Grenzwert in Abhängigkeit von der Anfangsreaktionszeit vorzugeben. Beispielsweise kann die Beendigung erfolgen, wenn sich die Reaktionszeit um mehr als 30 % gegenüber der anfänglichen Reaktionszeit erhöht hat.

Aufgrund der Entscheidungen der Steuereinheit über die "Untersuchungsstrategie" in Abhängigkeit vom Leistungsvermögen der zu untersuchenden Person erhält man auch beim Einsatz von ungeübtem Bedienungspersonal ein zuverlässiges Untersuchungsergebnis, das unter "standardisierten" Untersuchungsbedingungen gewonnen wird. Die erfindungsgemäße Vorrichtung hat dabei den zusätzlichen Vorteil, daß die Bedienung der Vorrichtung für das Bedienungspersonal drastisch vereinfacht wird, da die Bedienungsperson lediglich noch die Untersuchung starten muß.

Weiterbildungen der Erfindung sind in den Unteransprüchen angegeben.

Im Anspruch 2 ist ein vorteilhafter Ablauf der Darbietung der einzelnen Lichtpunkte gekennzeichnet. Die Steuereinheit läßt zunächst Lichtpunkte in einem zentralen 25°-Gesichtsfeldbereich mit einem Raster, d.h. einem Lichtpunktabstand, von ca. 6° aufleuchten und stellt die Anzahl der Defekte sowie den Verlauf der Reaktionszeit in Abhängigkeit von der Untersuchungsdauer fest.

Zeigt sich während dieses im Anspruch 2 gekennzeichneten Vortestes noch keine Ermüdung der zu untersuchenden Person, d.h. verlängert sich die Reaktionszeit noch nicht bzw. noch nicht wesentlich, so führt gemäß Anspruch 3 die Steuereinheit die Untersuchung in einem Haupttest in Abhängigkeit von der Zahl der Defekte, d.h. in Abhängigkeit von der Zahl der bei dem zu untersuchenden Auge angepaßter Intensität nicht gesehenen Lichtpunkte fort.

Gemäß einer vorteilhaften Ausgestaltung der Erfindung führt die Steuereinheit die Untersuchung nur dann in der im Anspruch 4 angegebenen Weise fort, wenn sich auch im Haupttest keine Ermüdungserscheinungen, d.h. kein Überschreiten des Grenzwertes der Reaktionszeit, einstellen.

In jedem Falle ist es besonders vorteilhaft, wenn die Steuereinheit als Kriterium für die Ermüdung der zu untersuchenden Person eine mittlere Reaktionszeit heranzieht (Anspruch 5). Hierdurch wird verhindert, daß die Untersuchung aufgrund einer nur kurzzeitigen Konzentrationsschwäche beendet wird. Dabei kann die Mittelung beispielsweise über fünf oder zehn Darbietungen erfolgen.

In den Ansprüchen 6 und 7 sind zusätzliche Kriterien für das Feststellen von Ermüdung der untersuchten Person angegeben. Ein zuverlässiges Anzeichen für Ermüdungserscheinungen ist es beispielsweise, wenn die zu untersuchende Person den Fixierpunkt bzw. die Fixiermarke beispielsweise im Mittelpunkt einer Perimeterschale nicht mehr fixiert (Anspruch 7). Weiterhin ist mit dem Auftreten von Ermüdungserscheinigungen oft das Geben falscher Antworten auf Fangfragen, d.h. beispielsweise das Geben der Antwort "gesehen" auch dann, wenn kein Lichtpunkt aufleuchtet, verbunden (Anspruch 6).

Eine im Anspruch 8 gekennzeichnete besonders vorteilhafte Ausgestaltung der Erfindung läßt es zu, die Untersuchung zu unterbrechen und nach einer Pause an der Stelle fortzuführen, an der Ermüdungserscheinungen aufgetreten sind. Hierzu wird das Untersuchungsergebnisses in Zuordnung zu einer bestimmten untersuchten Person gespeichert und nach einer Erholungspause erneut in die Vorrichtung zur Gesichtsfeldprüfung eingegeben. Die Steuereinheit startet dann den Untersuchungsablauf an der Stelle, an der unterbrochen worden ist, so daß nicht mehr durch langwierige "Vortests" die zu untersuchende Person erneut ermüdet wird, bevor mit neuen Untersuchungen begonnen wird.

Die Erfindung wird nachstehend anhand der Zeichnung exemplarisch beschrieben, in der zeigen:

Fig. 1 den prinzipiellen Aufbau einer erfindungsgemäßen Vorrichtung, und

Fig. 2 ein Ablaufdiagramm zur Erläuterung der von der Steuereinheit vorgegebenen Arbeitsweise einer erfindungsgemäßen Vorrichtung

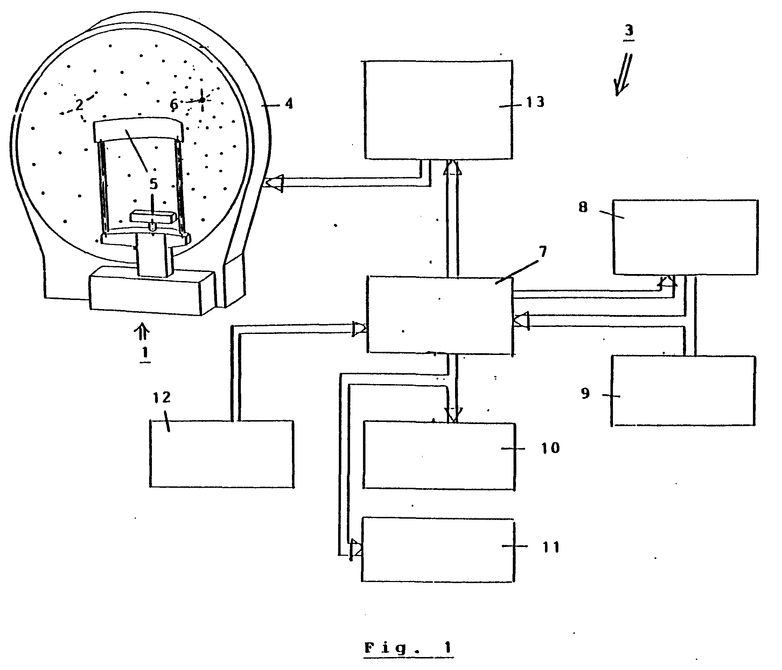

In Fig. 1 sind schematisch eine Einrichtung 1, in der über das Gesichtsfeld einer zu untersuchenden Person verteilte Lichtpunkte 2 aufleuchten, und ein Blockschaltbild einer Steuereinheit 3 dargestellt.

Die Einrichtung 1 weist eine halbkugelförmige Schale 4, die aufgeständert sein kann, und eine kombinierte Stirn- und Kopfstütze 5 auf. Auf der Innenfläche der Schale 4 befinden sich die Lichtpunkte 2, die beispielsweise Leuchtdioden (LED) sein können, sowie eine Fixationsmarke 6, die die zu untersuchende Person während der Gesichtsfeldprüfung fixieren muß.

Die Steuereinheit 3, die im Blockschaltbild dargestellt ist, weist einen Mikroprozessor 7 auf, der mit einem Schreib/Lese-Speicher (RAM) 8, einen Festwertspeicher (ROM oder EPROM) 9, einer Anzeigeeinheit 10, einem Befunddrucker 11 und einer Eingabeeinheit 12 verbunden ist. Ferner übernimmt der Mikroprozessor 7 über eine Ansteuerschaltung 13 die Ansteuerung der Leuchtdioden 2. Die Ansteuerschaltung 13 weist in bekannter Weise eine LED-Stromquelle, einen LED-Dekoder und eine LED-Frequenzansteuerung zur Variation der Intensität der einzelnen Leuchtdioden auf.

In dem Festwertspeicher 9 ist unter anderem ein Ablaufprogramm niedergelegt, gemäß dem die Steuereinheit 3 den Betrieb der erfindungsgemäßen Vorrichtung steuert. Beispielsweise läßt die Steuereinheit - wie im einzelnen in der DE-A-3 135 383 oder der DE-A-3 135 384 beschrieben - zunächst die Leuchtdioden 2 in einem zentralen Bereich des Gesichtsfeldes aufleuchten, variiert die Intensität der Leuchtdioden um die Intensität zu ermitteln, bei der die untersuchten Person einen Lichtpunkt "gerade noch" sieht und läßt anschließend in weiteren Untersuchungsschritten Dioden im äußeren Bereich des Gesichtsfeldes sowie noch nicht abgefragte Dioden im zentralen Bereich des Gesichtsfeldes aufleuchten. Dabei kann dem Aufleuchten eines Lichtpunktes bzw. einer Leuchtdiode 2 ein akustisches Aufmerksamkeitssignal vorausgehen; ferner können vereinzelt nach einem akustischen Aufmerksamkeitssignal keine Lichtpunkte aufleuchten, um zu testen, ob die untersuchte Person wahrheitsgemäß die Antwort "nicht gesehen" gibt.

In dem Schreib/Lese-Speicher 8 speichert die Steuereinheit 3 im wesentlichen die Antwort der untersuchten Person in Zuordnung zum Ort und zur Intensität des aufleuchtenden Lichtpunktes 2 sowie die jeweilige Reaktionszeit.

Das Ergebnis der Untersuchung, d.h. das Vorliegen und die Schwere von Gesichtsfelddefekten, kann beispielsweise über die Anzeigeeinheit 10, die ein Leuchtdiodenfeld aufweisen kann, angezeigt und mittels des Befunddruckers 11 ausgegeben werden.

Dieser allgemeine programmgesteuerte Ablauf einer Gesichtsfeldprüfung ist bekannt und muß deshalb nicht im einzelnen dargestellt werden. Bezüglich des allgemeinen Aufbaus der Vorrichtung wird beispielsweise auf das unter der Bezeichnung "Peritest" von der G. Rodenstock Instrumente GmbH, München, Deutschland, vertriebene Gerät verwiesen.

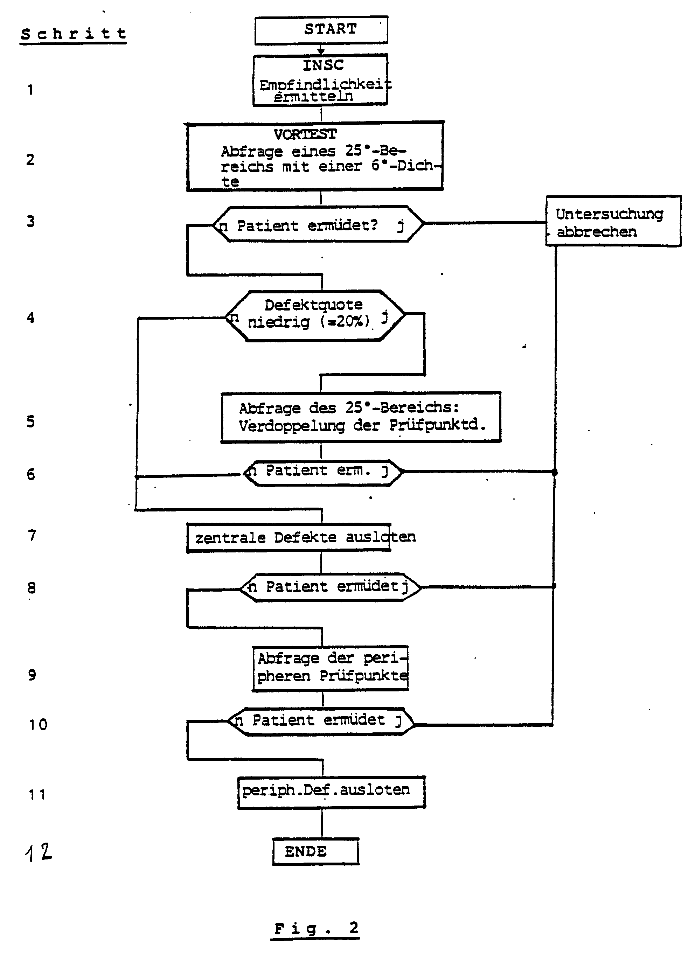

Fig. 2 zeigt ein Ablaufdiagramm, das den von der Steuereinheit der erfindungsgemäßen Vorrichtung bewirkten Ablauf zeigt.

In dem mit "INSC" bezeichneten Untersuchungsschritt 1 ermittelt die Steuereinheit die Empfindlichkeit des Auges der untersuchten Person, d.h. sie bestimmt die Intensität mehrerer ausgewählter Lichtpunkte, bei der die untersuchte Person den Lichtreiz "gerade noch" wahrnimmt. Diese Bestimmung bildet die Voraussetzung dafür, daß die Lichtpunkte bei dem auf den Schritt 1 folgenden Untersuchungsschritt in der der Empfindlichkeit des untersuchten Auges angepaßten Intensität wahrgenommen werden. Darüberhinaus wird in dem Schritt 1 ein grober Überblick über die Defektquote dadurch gewonnen, daß ca. 20 über das Gesichtsfeld der untersuchten Person verteilte Lichtpunkte aufleuchten, wobei sich diese Lichtpunkte hauptsächlich in einem zentralen 20°-Bereich des Gesichtsfelds befinden.

In dem mit "VORTEST" bezeichneten Untersuchungsschritt 2 läßt die Steuereinheit Lichtpunkte in einem 20°-Bereich des Gesichtsfelds der zu untersuchenden Person aufleuchten, wobei die einzelnen Lichtpunkte voneinander etwa einen Abstand von 6° haben. Um erkennen zu können, ob die zu untersuchende Person auch dann, wenn sie eigentlich nichts gesehen hat, die Antwort "gesehen" gibt, läßt die Steuereinheit in unregelmäßigen Abständen das Aufleuchten eines Lichtpunktes "ausfallen". Nur dann, wenn die zu untersuchende Person in diesen Fällen mit "nicht gesehen" antwortet, erlaubt die Untersuchung eine zuverlässige Aussage über das tatsächlich Gesichtsfeld der untersuchten Person.

Während des "VORTESTS" ermittelt die Steuereinheit laufend die Reaktionszeit der untersuchten Person, d.h. die Zeit, die zwischen dem Aufleuchten eines Lichtpunktes oder dem Beginn eines akustischen Aufmerksamkeitssignals und dem Geben der Antwort "gesehen" vergeht. Darbietungen, d.h. aufleuchtende Lichtpunkte, bei denen die Antwort "nicht gesehen" gegeben wird, bleiben bei der Ermittlung der Reaktionszeit außer betracht.

Nach Beendigung des "Vortests" wird in dem mit 3 bezeichneten "Entscheidungsschritt" von der Steuereinheit geprüft, ob die Reaktionszeit während des "Vortests" aufgrund von Ermüdungserscheinungen zu stark angewachsen ist.

Als Kriterium für Ermüdungserscheinungen kann beispielsweise ein Anwachsen der Reaktionszeit um mehr als 30 % gegenüber der ursprünglich ermittelten Reaktionszeit genommen werden. Natürlich können - beispielsweise in Abhängigkeit vom Alter, vom Intelligenzquotienten etc. der untersuchten Person - auch anderen Kriterien für Ermüdungserscheinungen, d.h. insbesondere auch andere Grenzen für die Reaktionszeit bzw. den Reaktionszeitzuwachs verwendet werden.

Stellt die Steuereinheit Ermüdung der untersuchten Person fest (Verzweigung j) so wird die Untersuchung beendet; die Steuereinheit speichert dann die ermittelten Daten in Zuordnung zur untersuchten Person.

Stellt die Steuereinheit dagegen noch keine Ermüdungserscheinungen fest (Verzweigung n), so ermittelt sie im Entscheidungsschritt 4 die Defektquote, d.h. die Zahl der nicht gesehenen Lichtpunkte. Liegt die Defektquote über 20 % (Verzweigung n), fährt die Steuereinheit mit der Auslotung der Defekte im zentralen 25°-Bereich fort (Schritt 7); diese Auslotung der Defekte im zentralen 25°-Bereich erfolgt durch nochmaliges Aufleuchten der bereits abgefragten Lichtpunkte mit erhöhter Intensität.

Ist dagegen die Defektquote niedrig (Verzweigung j), d.h. liegt sie unter 20 %, so startet die Steuereinheit eine nochmalige Abfrage des zentralen 25°-Bereichs, wobei die im Untersuchungsschritt 5 abgefragten Lichtpunkte 2 in den Lücken der im "VORTEST" (Schritt 2) abgefragten Lichtpunkte liegen. Hierdurch wird der zentrale Bereich bei Patienten mit geringer Defektquote mit erhöhter Prüfpunktdichte abgefragt. Dies ermöglicht in vorteilhafter Weise das Erkennen auch kleinstflächiger Gesichtsfeldausfälle.

Im Anschluß an diese Abfrage stellt die Steuereinheit fest, ob Ermüdungserscheinungen aufgetreten sind, oder ob nicht (Schritt 6). Beim Auftreten von Ermüdungserscheinungen bricht die Steuereinheit die Untersuchung ab und speichert die bis dahin erhaltenen Ergebnisse in Zuordnung zum jeweils untersuchten Patienten. Sind noch keine Ermüdungserscheinungen aufgetreten, so fährt die Steuereinheit nunmehr mit dem bereits beschriebenen Schritt 7 fort.

Gleichgültig mit welcher Vorgeschichte die Steuereinheit bis zum Schritt 7 gekommen ist, stellt sie nach Beendigung des Schrittes 7 im Schritt 8 erneut fest, ob die zu untersuchende Person ermüdet ist. Beim Auftreten von Ermüdungserscheinungen erfolgen ein Abbruch der Untersuchung und eine Speicherung der erhaltenen Ergebnisse, während wenn noch keine Ermüdungserscheinungen aufgetreten sind, im Schritt 9 die peripheren Lichtpunkte, d.h. die Lichtpunkte außerhalb des zentralen 25°-Bereichs abgefragt werden. Ist bei Beendigung dieses Untersuchungsschrittes der Patient noch nicht ermüdet, so erfolgt im Untersuchungsschritt 11 die Auslotung der peripheren Effekte, d.h. die Steuereinheit läßt die bereits überprüften Lichtpunkte nochmals mit höherer Intensität aufleuchten. Nach Beendigung dieses Untersuchungsschritts beendet die Steuereinheit im Schritt 12 die Untersuchung und speichert die Ergebnisse in Zuordnung zu der untersuchten Person.

Die erfindungsgemäße Steuereinheit steuert nicht nur die vorstehend beschriebenen Funktionsabläufe, wie beispielsweise das Aufleuchten von über das Gesichtsfeld verteilten punktförmigen Lichtreizen, sondern auch die jeweilige Intensität der Lichtreize, und speichert die Antwort der untersuchten Person in Zuordnung zum Ort des Lichtreizes. Hierzu kann die Steuereinheit auf beliebige Art aufgebaut sein, beispielsweise aus handelsüblichen Logikschaltungen. Bevorzugterweise besteht die Steuereinheit für die erfindungsgemäße Vorrichtung zur Gesichtsfeldprüfung aus einem Mikroprozessor mit dem üblichen Peripherieeinheiten, wie Schreib/Lese-Speichern (RAM), zum Speichern der Reaktionszeit, der Antworten etc., Festwertspeichern (ROM oder EPROM), in denen beispielsweise der erfindungsgemäße Funktionsablauf niedergelegt ist, Ein- und Ausgabeeinheiten, die unter anderem eine Taste umfassen können, mit der die untersuchte Person die Antwort "gesehen" durch Drücken gibt. Die Ausgabeeinheiten können weiterhin eine Leuchtdioden-Anzeigeeinheit aufweisen, mit der die Anzahl der ermittelten Defekte im Gesichtsfeld sowie die Schwere der Defekte der Bedienungsperson angezeigt werden.

Während der Gesichtsfeldprüfung ermittelt die Steuereinheit laufend die Reaktionszeit der untersuchten Person, führt ferner beispielsweise die Mittelung der Reaktionszeit über eine bestimmte Zahl von Darbietungen aus und speichert die einzelnen Antworten. In den einzelnen Entscheidungsschritten prüft die Steuereinheit anhand der ermittelten Reaktionszeit, ob Ermüdungserscheinungen aufgetreten sind und bricht gegebenenfalls - wie vorstehend beschrieben - die Untersuchung ab.

Der Aufbau der Vorrichtung zur Gesichtsfeldprüfung, deren Funktion durch die vorstehend beschriebenen Steuereinheit gesteuert wird, kann in beliebiger Weise ausgeführt sein. Vorrichtungen, die in Verbindung mit der erfindungsgemäß ausgebildeten Steuereinheit verwendbar sind, sind beispielsweise in der US-A 3 883 235 beschrieben. Besonders vorteilhaft kann jedoch eine Vorrichtung verwendet werden, die als Einrichtung, in der über das Gesichtsfeld einer zu untersuchenden Person verteilte Lichtpunkte aufleuchten, eine aufgeständerte Halbkugelschale aufweist, über deren Innenfläche Leuchtdioden, die jeweils einen Lichtpunkt bilden, verteilt sind. Die Leuchtdioden werden in bekannter Weise von der Steuereinheit angesteuert, die den Aufleucht-Zeitpunkt und die Intensität der einzelnen Lichtpunkte bestimmt. Eine derartige Einrichtung wird beispielsweise bei der unter dem Namen "Peritest" vertriebenen Vorrichtung zur Gesichtsfeldprüfung der G. Rodenstock Instrumente GmbH, München, Deutschland verwendet.

Da bei Kenntnis der vorstehend beschriebenen Funktion der erfindungsgemäßen Vorrichtung gemäß einem der Ansprüche 1 bis 8 der Aufbau einer derartigen Vorrichtung ohne weiteres möglich ist, soll an dieser Stelle auf Einzelheiten der Schaltungsausführung sowie des restlichen Aufbaus der erfindungsgemäßen Vorrichtung zur Gesichtsfeldprüfung verzichtet werden.

**Patentansprüche**

1. Vorrichtung zur Gesichtsfeldprüfung mit einer Einrichtung (1), in der über das Gesichtsfeld einer zu untersuchenden Person verteilte Lichtpunkte (2) aufleuchten, und mit einer Steuereinheit (3), die die Verteilung und die zeitliche Abfolge der dargebotenen Lichtpunkte steuert, und die ein Eingabeelement aufweist, mittels dem die zu untersuchende Person die Antwort "gesehen" bzw. "nicht gesehen" jeweils durch Auslösen oder Nichtauslösen eines Signals in die Steuereinheit eingibt, wobei die Steuereinheit laufend die Reaktionszeit der zu untersuchenden Person zwischen dem Aufleuchten eines Lichtpunkts und dem Geben der Antwort "gesehen" ermittelt, dadurch gekennzeichnet, daß die Steuereinheit so ausgebildet ist, daß sie beim Überschreiten eines vorgegebenen Grenzwertes der Reaktionszeit die Darbietung der Lichtpunkte beendet, nachdem die Untersuchung des beim Überschreiten des Grenzwertes untersuchten Gesichtsfeldbereichs abgeschlossen ist.

2. Vorrichtung nach Anspruch 1, dadurch gekennzeichnet, daß die Steuereinheit so ausgebildet ist, daß sie in einem Vortest zunächst die Lichtpunkte in einem zentralen 25°-Bereich des Gesichtsfeldes mit einem Raster von ca 6° aufleuchten läßt, und nur dann, wenn sich die Reaktionszeit am Ende der Darbietung dieser Lichtpunkte nicht wesentlich verlängert hat, weitere Lichtpunkte aufleuchten läßt.

3. Vorrichtung nach Anspruch 2, dadurch gekennzeichnet, daß die Steuereinheit so ausgebildet ist, daß sie sofern der Grenzwert für die Reaktionszeit im Vortest nicht überschritten worden ist, im Anschluß an den Vortest in einem Haupttest,
(a) wenn die Anzahl der nicht wahrgenommenen Lichtpunkte groß ist, die Tiefe der Gesichtsfelddefekte auslotet,
(b) wenn die Zahl der nicht wahrgenommenen Lichtpunkte klein ist, Lichtpunkte in den Lücken der dargebotenen Lichtpunkte aufleuchten läßt.

4. Vorrichtung nach Anspruch 3, dadurch gekennzeichnet, daß die Steuereinheit so ausgebildet ist, daß sie sofern der Grenzwert für die Reaktionszeit im Haupttest nicht überschritten worden ist, im Anschluß an den Haupttest,
(a) wenn die Zahl der nicht wahrgenommenen Lichtpunkte groß ist, Lichtpunkte außerhalb des zentralen 25°-Gesichtsfeldbereichs darbietet,
(b) wenn die Zahl der nicht wahrgenommenen Lichtpunkte klein ist, die Tiefe der im zentralen 25°-Gesichtsfeldbereich vorhandenen Defekte auslotet, und anschließend, sofern der Grenzwert für die Reaktionszeit noch nicht überschritten worden ist, Lichtpunkte außerhalb des zentralen 25°-Gesichtsfeldbereichs darbietet, und ggfl. anschließend deren Defekttiefe auslotet.

5. Vorrichtung nach einem der Ansprüche 1 bis 4, dadurch gekennzeichnet, daß die Steuereinheit so ausgebildet ist, daß sie die Reaktionszeit über mehrere Darbietungen mittelt.

6. Vorrichtung nach einem der Ansprüche 1 bis 5,

dadurch gekennzeichnet, daß die Steuereinheit so ausgebildet ist, daß sie auch dann, wenn die zu untersuchende Person zu häufig auch beim beabsichtigen Nichtaufleuchten eines Lichtpunktes die Antwort "gesehen" gibt, die Darbietung der Lichtpunkte beendet, nachdem die Untersuchung des geraden untersuchten Gesichtsfeldbereiches abgeschlossen ist.

7. Vorrichtung nach einem der Ansprüche 1 bis 6,

dadurch gekennzeichnet, daß die Steuereinheit so ausgebildet ist, daß sie auch dann, wenn die zu untersuchende Person zu häufig die Fixationsmarke nicht mehr fixiert, die Darbietung der Lichtpunkte beendet, nachdem die Untersuchung des gerade untersuchten Gesichtsfeldbereiches abgeschlossen ist.

8. Vorrichtung nach einem der Ansprüche 1 bis 7,

dadurch gekennzeichnet, daß die Steuereinheit so ausgebildet ist, daß sie bei Beendigung der Untersuchung die Untersuchungsergebnisse in Zuordnung zur untersuchten Person speichert, so daß die Untersuchung zu einem späteren Zeitpunkt an der Stelle fortsetzbar ist, an der die Untersuchung wegen Ermüdung unterbrochen worden ist.

**Claims**

1. Apparatus for testing the field of view, comprising means (1) wherein light spots (2) distributed over the field of view of a person to be examined flash up, and including a controller (3) to control the distribution and the chronological sequence of the offered light spots, which controller includes an entry element for use by the person to be examined to enter into the controller the "seen", or "not seen" response, triggering or not triggering, respectively, a signal, wherein said controller continuously determines the response time of the person to be examined, which lapses between the time at which a light spot flashes up and the time by which the "seen" response is entered, characterized in that the controller is so designed that it terminates the presentation of the light spots when a defined response time limit value will have been exceeded upon termination of the examination of the zone in the field of view which was under examination by the time when the limit value was exceeded.

2. Apparatus according to claim 1, characterized in that the controller is so designed that, in a preliminary test, it causes initially the light spots to flash up in a pattern of roughly 6° within a central 25° zone of the field of view, and that it causes further light spots to flash up only when the response time lapsed by the end of the presentation of these light spots will not have been substantially extended.

3. Apparatus according to claim 2, characterized in that the controller is so designed that if the response time limit value will not have been exceeded in the preliminary test, in a principal test following the preliminary test,

(a) it probes the depth of the field of view effects when the number of light spots not perceived is great,

(b) or causes light spots to flash up in the intervals between the presented light spots when the number of light spots not perceived is small.

4. Apparatus according to claim 3, characterized in that the controller is so designed that that if the response time limit value will not have been exceeded in the principal test, subsequently to the principal test,

(a) it presents light spots outside the central 25° zone in the field of view if the number of light spots not perceived is great,

(b) or probes the depth of the defects manifest in the central 25° zone in the field of view when the number of light spots not perceived is small, and subsequently presents light spots outside the central 25° zone in the field of view if the response time limit value will not have been exceeded, whenever applicable with subsequent probing of the depth of these defects.

5. Apparatus according to any of claims 1 through 4, characterized in that the controller is so designed that it ascertains a mean value of the response times established over several presentations.

6. Apparatus according to any of claims 1 through 5, characterized in that the controller is so designed that even if the person to be examined will too frequently enter the "seen" response also in the event of intentional non-flash-up of a light spot, it terminates the presentation of the light spots when the examination of the zone in the field of view then under examination will have been finished.

7. Apparatus according to any of claims 1 through 6, characterized in that the controller is so designed it even if the person to be examined will too frequently no longer gaze fixedly at the fixing marker, it terminates the presentation of the light spots when the examination of the zone in the field of view then under examination will have been finished.

8. Apparatus according to any of claims 1 through 7, characterized in that the controller is so designed that upon termination of the examination it will store the results of the examination with assignment to the person examined such that the examination may be continued later on at the point where it had been interrupted for reasons of fatigue.

## Revendications

1. Installation pour l'examen du champ de vision, comprenant des moyens (1) dans lesquels des points lumineux (2) s'allument qui sont répartis sur le champ de vision d'une personne à examiner, ainsi qu'un dispositif de commande (3) pour contrôler la répartition et l'ordre chronologique des points lumineux présentés et de plus comprenant un élément d'entrée à l'utilisation par la personne à examiner pour l'entrée, dans ledit dispositif de commande, de la réponse "vu" ou "pas vu" par le déclenchement ou non-déclenchement d'un signal, ledit dispositif de commande établissant en continu le temps de réaction de la personne à examiner qui s'écoule entre l'éclairage d'un point lumineux et l'entrée d'une réponse,

caractérisée en ce que ledit dispositif de commande est si formé qu'il termine la présentation des points lumineux quand le temps de réaction dépasse une valeur limite définie, après la fin de l'examen de la zone du champ de vision respectivement examinée au dépassement de ladite valeur limite.

2. Installation selon la Revendication 1,

caractérisée en ce que ledit dispositif de commande est si formé qu'au cours d'un examen préalable, il fait d'abord les points lumineux s'allumer au dedans d'une zone centrale à 25° à une trame de 6° environ, et fait des autres points lumineux s'allumer seulement quand, à la fin de leur présentation, le temps de réaction n'était essentiellement plus prolongé.

3. Installation selon la Revendication 2,

caractérisée en ce que le dispositif de commande est si formé qu'à condition que la valeur limite du temps de réaction ne soit pas dépassée au cours d'examen préalable,

(a) il sonde la profondeur des effets du champs de vision quand le nombre des points lumineux non-perçus est grand,

(b) ou fait des points lumineux s'allumer dans les interstices des points lumineux présentés quand le nombre des points lumineux non-perçus est petit, l'un et l'autre au cours d'un examen principal à la suite de l'examen préalable.

4. Installation selon la Revendication 3,

caractérisée en ce que le dispositif de commande est si formé qu'à condition que la valeur limite du temps de réaction ne soit dépassée au cours de l'examen principal, à la suite de l'examen principal, il

(a) présente des points lumineux au dehors de la zone centrale à 25° du champs de vision quand le nombre des points lumineux non-perçus est grand,

· (b) ou sonde la profondeur des troubles qui se manifestent dans la zone centrale à 25° du champs de vision, quand le nombre des points lumineux non-perçus est petit, et présente, à condition que la valeur limite du temps de réaction ne soit déjà dépassée, des points lumineux au dehors de la zone centrale à 25° du champs de vision, et, le cas échéant, sonde ci-après leur profondeur de trouble.

5. Installation selon quelconque des Revendications 1 à 4,

caractérisée en ce que le dispositif de commande est si formé qu'il calcule la moyenne des temps de réaction mesurés dans plusieurs présentations.

6. Installation selon quelconque des Revendications 1 à 5,

caractérisée en ce que le dispositif de commande est si formé qu'il termine la présentation desdits points lumineux, après la terminaison de l'examen de la zone du champs de vision respectivement examinée, même quand la personne à examiner donne la réponse "vu" trop fréquemment aussi au cas de non-éclairage intentionnel d'un point lumineux.

7. Installation selon quelconque des Revendications 1 à 6,

caractérisée en ce que le dispositif de commande est si formé qu'il termine la présentation desdits points lumineux, après la terminaison de l'examen de la zone du champs de vision respectivement examinée, même quand la personne à examiner non regarde plus fixement la marque de fixation trop fréquemment.

8. Installation selon quelconque des Revendications 1 à 7,

caractérisée en ce le dispositif de commande est si formé qu'à terminaison de l'examen, il met en mémoire les résultats de l'examen en coordination avec la personne examinée d'une façon que la continuation de l'examen soit possible ultérieurement au point où l'examen était interrompu à cause de fatigue.

Fig. 1

Schritt

```
                    ┌─────────────┐
                    │    START    │
                    └──────┬──────┘
                           │
                    ┌──────┴──────┐
 1                  │    INSC     │
                    │Empfindlichkeit│
                    │  ermitteln  │
                    └──────┬──────┘
                    ┌──────┴──────────────┐
                    │      VORTEST        │
 2                  │Abfrage eines 25°-Be-│
                    │reichs mit einer 6°-Dich-│
                    │te                   │
                    └──────┬──────────────┘
                                                      ┌──────────────┐
 3           <n  Patient ermüdet?   j>────────────────│ Untersuchung │
                                                      │  abbrechen   │
                                                      └──────┬───────┘
              ┌──────────────────────────┐
 4           <n niedrig (≈20%)  j  Defektquote>
              │
              │     ┌────────────────────────────┐
 5                  │Abfrage des 25°-Bereichs:   │
                    │Verdoppelung der Prüfpunktd.│
                    └─────────┬──────────────────┘
 6           <n Patient erm. j>──────────────────────────────┐
                                                             │
 7           │ zentrale Defekte ausloten │                   │
 8           <n Patient ermüdet j>───────────────────────────┤
                    ┌───────────────────┐
 9                  │Abfrage der peri-  │
                    │pheren Prüfpunkte  │
                    └─────────┬─────────┘
 10          <n Patient ermüdet j>──────────────────────────┘
 11          │ periph.Def.ausloten │
                    ┌─────────┐
 12                 │  ENDE   │
                    └─────────┘
```

Fig. 2